# EUROPÄISCHE PATENTSCHRIFT

(11) **EP 0 749 452 B1**
(45) Veröffentlichungstag und Bekanntmachung des Hinweises auf die Patenterteilung: **17.07.2002**
(21) Anmeldenummer: 95912219.3
(22) Anmeldetag: 07.03.1995
(51) Int. Cl.: C08G 69/10, C08G 73/06, G01N 33/545, C07H 17/04

(54) **POLYMERKONJUGIERTE MALONSÄUREDERIVATE UND DEREN VERWENDUNG ALS ARZNEITMITTEL UND ALS DIAGNOSTIKA**
POLYMER-CONJUGATED MALONIC ACID DERIVATIVES AND THEIR USE AS MEDICAMENTS AND DIAGNOSTIC AGENTS
DERIVES D'ACIDE MALONIQUE CONJUGUES A UN POLYMERE ET LEUR UTILISATION COMME MEDICAMENTS OU AGENTS DIAGNOSTIQUES

(30) Priorität: 11.03.1994 DE 4408248
(43) Veröffentlichungstag der Anmeldung: 27.12.1996
(73) Patentinhaber: Glycorex AB, 223 70 Lund (SE)
(72) Erfinder: TOEPFER, Alexander, D-65830 Kriftel (DE); KRETZSCHMAR, Gerhard, D-65760 Eschborn (DE); BARTNIK, Eckart, D-65205 Wiesbaden (DE); SCHMIDT, Wolfgang, D-65929 Frankfurt (DE); HÖRSCH, Brigitte, D-65830 Kriftel (DE)
(74) Vertreter: Henriksson, Dan Ragnar Mikael
(86) Internationale Anmeldenummer: EP9500844
(87) Internationale Veröffentlichungsnummer: WO9524437

(56) Entgegenhaltungen:
- EP-A- 0 406 473
- WO-A-93/14127
- DD-A- 273 443
- PATENT ABSTRACTS OF JAPAN vol. 13 no. 372 (C-627) [3720] ,17.August 1989 & JP,A,01 127039 (CHUICHI HIRAYAMA)

## Beschreibung

Die vorliegende Erfindung betrifft polyvalente Malonsäurederivate mit antiadhäsiven Eigenschaften auf Polymerbasis, die in vivo weder in ihrer Gesamtheit noch in Form von Abbauprodukten Unverträglichkeitsreaktionen hervorrufen, ihr Herstellungsverfahren und ihre Verwendung als Arzneimittel und Diagnostika.

Die auf der Oberfläche eukaryontischer Zellen befindlichen Glycoconjugate stellen spezifische Bindungsepitope für Transmembran-Proteine dar, welche man als Selektine bezeichnet. Diese Selektine, welche sowohl im Endothel als auch auf den verschiedenen zirkulierenden Zellen des hämatolymphischen Systems vorkommen, gehen spezifische Wechselwirkungen mit Kohlenhydrat-Epitopen (Liganden) des jeweils anderen Zelltyps ein (K.A. Karlsson, TIPS 12, (1991), 265-272).

Synthetische Analoga der Selektin-Liganden sind daher vielversprechende Kandidaten für Antiphlogistika und Antikoagulantien. Auch als Erkennungsdomänen für Viren, Bakterien und Toxine spielen Kohlenhydratliganden eine entscheidende Rolle und damit auch bei der Einleitung der entsprechenden Krankheiten (Prophylaxe, Diagnostik oder Therapie von bakteriellen und viralen Infektionen, entzündlichen Erkrankungen, rheumatische Arthritis, Allergien, Nachinfarktsyndrom, Schock, Schlaganfall, akute und chronische Organabstoßung, Vasculitis, Sepsis, Schocklunge, usw.).

Da Krebszellen ein anderes Muster dieser Kohlenhydratstrukturen aufweisen als gesunde Zellen, ermöglicht dies die Verwendung dieser Mimetika einerseits als Marker, andererseits zur Bekämpfung von Tumorzellen, insbesondere bei metastasierenden Tumoren (S.I. Hakomori, Cancer Cells, December 1991, Vol.3, No. 12).

Von besonderer Bedeutung als Mediatoren der Zelladhäsion sind sialylierte und / oder fucosylierte Kohlenhydrate wie z.B. Sialyl-Lewis X und Sialyl-Lewis-A (Lowe et al, Cell, 63, 475-85, 1990).

Mit dem Ziel einer Vereinfachung der sehr aufwendigen Synthesen dieser Verbindungen bei Erhaltung oder Verbesserung ihrer Bindungsaffinitäten zu den entsprechenden Selektinen wurden bereits einfachere Strukturen vorgeschlagen und teilweise synthetisiert. So wurde z.B. Neuraminsäure durch Milch- oder Glycolsäure und/oder Fucose durch Glycerin bzw. Trifluormethylfucose und/oder N-Acetylglucosamin durch Glucosamin oder Glucose ersetzt (PCT/US 92/09870). Eine Substitution von Neuraminsäure durch Sulfat oder Phosphat ist ebenfalls beschrieben (PCT/CA 92/00245). Beschrieben ist außerdem eine Substitution von Glucosamin durch eine Kette von mindestens 2 Kohlenstoffatomen (WO 92/18610).

Die Therapie entzündlicher Erkrankungen mit freien Oligosacchariden, die anstelle der natürlichen Liganden an Rezeptoren binden sollen, scheitert an den sehr hohen Mengen des zu verabreichenden Oligosaccharids, da die Affinität zwischen Rezeptor und Oligosaccharid gering ist (K_{D} ~ 10⁻⁴M bei der Wechselwirkung zwischen einem monovalenten Galactosid und dem entsprechenden Lectin D. T. Connolly et al. J. Biol. Chem. 257, 939, (1982)).

Divalente Strukturen mit z.T. besserer Bindung an den jeweiligen Rezeptor werden von Wong et al. (J. Am. Chem. Soc. 115, 7549 (1993)) und in der US 5,254,676 beschrieben.

Es ist außerdem bekannt, daß eine erhöhte Wechselwirkung zwischen Rezeptor und Ligand durch die Kopplung mehrerer Liganden auf einer Oberfläche erreicht wird. Am Beispiel des Virusproteins Hemaglutinin, das an Neuraminsäure auf der Zelloberfläche bindet, konnte gezeigt werden, wie durch Verwendung eines Polymers dieser polyvalente Effekt sich signifikant auf die Wechselwirkung Ligand-Rezeptor auswirkt ( monovalent K_{D} = 2x20⁻⁴M, polyvalent K_{D} = 3x10⁻⁷M, A. Spaltenstein et al. J. Am. Chem. Soc. 113, 686 (1991)).

Als Oberflächen wurden bisher Liposomen ( N. Yamazaki, Int. J. Biochem. 24, 99 (1991); WO 91/19501; WO 91/19502), Polyacrylamide ( R.C. Rathi et al. J. Polym. Sci.: Part A: Polym. Chem. 29, 1895 (1991), S.-I. Nishimura et al. Macromolecules 24, 4236 (1991)), Polylysin oder sulfatierte Polysaccharide verwendet. Diese polyvalenten Strukturen haben den Nachteil entweder in vivo nur wenig stabil oder durch Abbau in toxische Metaboliten in vivo unverträglich zu sein. Bei Polylysin oder sulfatierten Polysacchariden treten unspezifische Wechselwirkungen mit Zelloberflächenstrukturen auf.

DD-A-273 443 beschreibt ein Verfahren zur Herstellung von 3'-Carboxyl-substituierten Oligodes oxynucleotiden unter Verwendung träger gebundener Spacer.

Aufgabe der vorliegenden Erfindung ist es, polyvalente Malonsäurederivate auf Polymerbasis mit antiadhäsiven Eigenschaften bereitzustellen, die in vivo weder in ihrer Gesamtheit noch in Form von Abbauprodukten Unverträglichkeitsreaktionen hervorrufen. Diese sollen gegenüber den natürlichen Liganden eine vergleichbare oder stärkere Wechselwirkung mit dem Rezeptor aufweisen und sich im Vergleich mit den näturlichen Kohlenhydrat-Liganden einfacher und in größeren Mengen herstellen lassen.

Es ist ferner die Aufgabe der vorliegenden Erfindung, auf Basis dieser polymeren und polyvalenten Malonsäurederivate Arzneimittel zur Therapie oder Prophylaxe und Mittel zur Diagnose von Krankheiten verfügbar zu machen, bei welchen bakterielle oder virale Infektionen, metastasierende Tumore oder endzündliche Prozesse beteiligt sind.

Diese Aufgabe wird erfindungsgemäß gelöst durch eine Verbindung bestehend aus einer Polyaminosäure, welche über jeweils einen Spacer der Formel I,

-[-Q¹-(CH₂)ₚ-Q²-]- I,

worin
- Q¹: -CH₂- oder 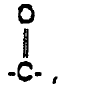
- Q²: -NH- oder 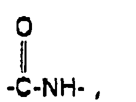
- p: eine Zahl von 1 bis 6 bedeuten,
mit wenigstens einem Rest der Formel II 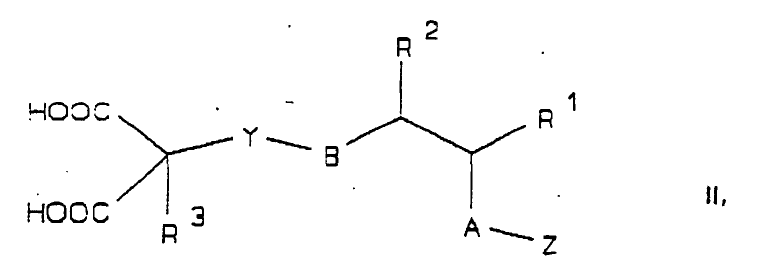 in der
- R¹ und R²: zu einem sechsgliedrigen Carbo- oder Heterocyclus mit mindestens einem der Ring-substituenten R⁴, R⁵ und R⁶ gehören und
- R³: H, (CH₂)ₘX oder CH₂O(CH₂)ₘX¹ bedeutet,
- A und B: unabhängig voneinander O, S, NH, HN-CO, OC-NH, O-CO, OC-O, NH-CO-O, O-CO-NH, S-CO, SC-O, O-CS-S, S-CS-O, NH-CS-S, S-CS-NH oder CH₂ bedeuten und
- Z: eine Pyranose, sine Furanose, einen offenkettigen Polyalkohol darstellt oder Y-X⁶ bedeutet,
- Y: -O-(CX²X³)ₙ, -(CX²X³)ₙ, -CH₂-(CX²X³)⁻ₙ ,wobei
- R⁴, R⁵ und R⁶: unabhängig voneinander H, OH, -O-(CH₂)_{q}X⁴, CH₂O(CH₂)_{q}X⁵ oder HNC(O)CH₃ und
- X, X¹, X² und X³: unabhängig voneinander H, NH₂, COOH, OH, CH₂OH, CH₂NH₂, C₁-C₂₀-Alkyl oder C₆-C₁₀-Aryl,
- X⁴ und X⁵: unabhängig voneinander -NH- oder -O- und
- X⁶: OH oder 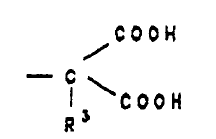 und
- m, n und q: unahängig voneinander eine Zahl von 1 bis 20 bedeuten, über einen der Ringsubstituenten R⁴, R⁵ oder R⁶ verknüpft ist, wobei der Belegungsgrad der Polyaminosäure mit dem Rest gemäß Formel II via Spacer zwischen 0.5 und 50 Mol% liegt.

Vorzugsweise ist die Polyaminosäure Polysuccinimid, Polyaspartamid, Polyglutamat oder Polylysinfumaramid, oder die Polyaminosäure ist ein Copolymer aus Polysuccinimid und Polyaspartamid, ein Copolymer aus Polyhydroxyethylaspartamid und Polyaspartamid oder ein Copolymer aus Polysuccinimid, Polyaspartamid und Polyhydroxyethylaspartamid.

Vorzugsweise zeichnet sich die erfindungsgemäße Verbindung dadurch aus, daß in dem Rest der Formel II R1 und R2 zu einem substituierten Tetrahydropyranring gehören,
- A und B: O,
- z: eine -Fucopyranosyl-Gruppe und
- Y: (CX²X³)ₙ bedeuten, wobei
- R³, X² und X³: H bedeuten und n für 5 steht.

Vorzugsweise bedeuten die Substituenten des Tetrahydropyranrings R⁴ und R⁵ jeweils H, der im Tetrahydropyranring zwischen dem Ringheteroatom O und der Ringsubstituenten -A-Z liegende Substituent R6 -CH₂O(CH₂)_{q}X⁵ und X⁵ -NH-; besonders bevorzugt steht q für 2.

Vorzugsweise ist der Tetrahydropyranring eine Pyranose.

Besonders bevorzugt ist die Pyranose N-Acetyl-D-glucosamin.

Vorzugsweise ist die Verbindung gemäß der vorliegenden Erfindung auch dadurch gekennzeichnet, daß
- R¹ und R²: zu einem sechsgliedrigen Carbocyclus gehören,
- A-Z: 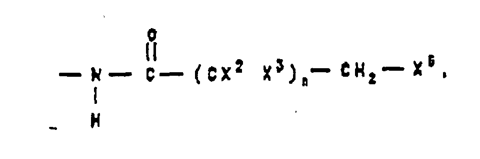
- -B-Y-: 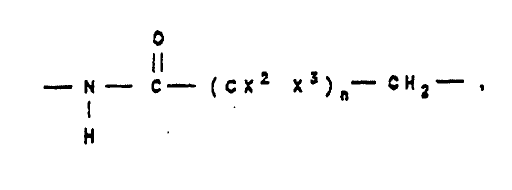
R³ und X² H, X³ und X⁶ OH und n 4 bedeuten, oder wahlweise dadurch gekennzeichnet ist, daß
- X⁶:

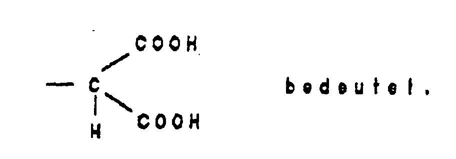

Die eingangs gestellte Aufgabe wird ferner durch ein Verfahren zur Herstellung der oben beschriebenen Verbindung gelöst, welches sich dadurch auszeichnet, daß die kovalente Verknüpfung des Polymers mit einer Verbindung der Formel III 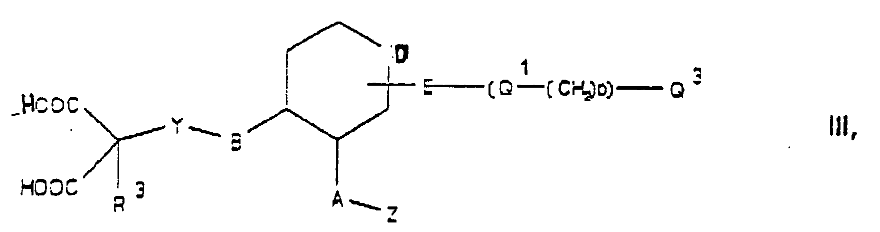 worin
- D: ·NH-, -O- oder -CH₂-,
- E: -O(CH₂)_{q}NH-, -O(CH₂)_{q}O-, -CH₂O(CH₂)_{q}NH- oder -CH₂O(CH₂)_{q}O- und
- Q³: -OH, -NH₂ oder 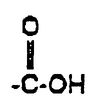 bedeuten und
die übrigen Variablen die vorgenannten Bedeutungen haben, durch Reaktion zwischen einer reaktiven und einer aktivierten Gruppe erfolgt.

Die Aufgabe wird ferner gelöst durch ein Arzneimittel, enthaltend die Verbindung gemäß der vorliegenden Erfindung und gegebenfalls pharmazeutische Träger.

Die Verbindung gemäß der vorliegenden Erfindung eignet sich vorteilhaft zur Herstellung eines Arzneimittels zur Therapie oder Prophylaxe von Krankheiten, bei welchen bakterielle oder virale Infektionen, entzündliche Prozesse oder metastasierende Tumoren involviert sind.

Die erfindungsgemäße Verbindung ist vorteilhaft auch geeignet zur Herstellung eines Mittels zur Diagnose von Krankheiten, bei welchen bakterielle oder virale Infektionen, entzündliche Prozesse oder metastasierende Tumoren involviert sind.

Die Erfindung wird im folgenden detailliert beschrieben, insbesondere in ihren bevorzugten Ausführungsformen:

Das polyvalente Malonsäurederivat auf Polymerbasis ist physiologisch verträglich und hat vorzugsweise ein Molekulargewicht von <70 kD. Gut verträglich bedeutet, daß durch die Verbindung keine Unverträglichkeitsreaktionen hervorgerufen werden. Das Malonsäurederivat und das Polymer alleine dürfen in vivo nicht schädigend für den Organismus sein, sie dürfen also nicht hämolytisch, nicht antigen und nicht thrombogen wirken.
Das polyvalente Malonsäurederivat ist physiologisch abbaubar, d.h. es ist in vivo als ganzes Molekül ausscheidbar oder in vivo zu kleinen physiologisch verträglichen Bausteinen abbaubar, um in dieser Form ausscheidbar (biodegradabel) zu sein. Dies gilt insbesondere für das Polymer.
Über die Abbaubarkeit in vivo wird die Verweildauer am jeweiligen Rezeptor und damit die Wirkzeit gesteuert. So kann z. B. über die Wahl der Polymerbausteine und deren Verknüpfung die Abbaubarkeit gesteuert werden. Bei Überschuß der Verbindung kann so eine Akkumulation im Organismus verhindert werden.

Die Begriffe Rezeptor und Ligand werden wie folgt definiert:
Ein Rezeptor ist ein Makromolekül auf der Oberfläche der Zelle, welches aufgrund seiner Konfiguration eine Bindungsstelle zur spezifischen Bindung einer, selten mehrerer Signalsubstanzen besitzt. Dies Signalsubstanz wird als Ligand bezeichnet. Ziel der Bindung des Liganden an den Rezeptor ist die Informationsübertragung in die Zelle und anschließende Zellstoffwechseländerung.

Der Belegungsgrad des Polymers mit dem Malonsäurederivat gibt den prozentualen Anteil der Repetiereinheiten des Polymers, an die das Malonsäurederivat geknüpft ist, bezogen auf die Gesamtheit der Repetiereinheiten an.

Grundsätzlich erfolgt die Synthese durch kovalente Knüpfung des Malonsäurederivats über einen Spacer an ein Polymer.

Der Spacer ist eine physiologisch verträgliche, natürliche oder synthetische Verbindung, die die Aufgabe der räumlichen Trennung von Malonsäurederivat und Polymer hat. Diese räumliche Trennung ist notwendig, um eine optimale Zugänglichkeit des Liganden für den Rezeptor zu gewährleisten.
Um eine Kopplung des Spacers an das Malonsäurederivat zu gewährleisten, muß der Spacer bifunktionell sein.

Die Herstellung von biodegradablem, hydrophilen Polymer erfolgt nach dem Fachmann bekannten Verfahren. Diese sind z. B. beschrieben in: H.G. Elias, Makromoleküle, Bd. 1 und 2, Hüthig & Wepf Verlag, Basel, Schweiz, 1991/92 oder D. Braun, H. Cherdron, W. Kern, Praktikum der Makromolekularen Organischen Chemie, Hüthig Verlag 1979.
Polymere, die in ihrer Funktion als Träger Bestandteil des polyvalenten Malonsäurederivats sind und diagnostische oder therapeutische Verwendung finden sollen, müssen grundsätzlich eine Reihe von Anforderungen erfüllen, um physiologisch gut verträglich zu sein:
- keine Immunantwort zu induzieren und
- um unspezifische Wechselwirkungen und Kumulation im Retikoendothelialen System zu vermeiden.

Definitionsgemäß besteht das Polymer aus wenigstens zwei gleichen oder veschiedenen Monomerbausteinen, die linear oder verzweigt miteinander verknüpft sind und eine Molekulargewichtsverteilung aufweisen können.

Das Polymer ist vorzugsweise eine Polyaminosäure als Polyamid oder -anhydrid verknüpft mit einem Molekulargewicht kleiner oder gleich 70 kD. Vorzugsweise hat das Polymer eine Mindestgröße von 2 kD, um im Vergleich zu niedermolekularen Trägern eine erhöhte Verweilzeit im Blut zu erzielen.

Für die Herstellung von Malonsäurederivaten auf Polymerbasis besonders bevorzugt geeignete Polyaminosäuren sind Polyaspartamide, Polysuccinimide, Polyglutamate und Polylysinfumaramide sowie deren Copolymere.

Der Belegungsgrad des Polymers mit dem Malonsäurederivat via Spacer liegt zwischen 0.5 und 50 Mol%, vorzugsweise zwischen 2-25 Mol%.

Das Polymer kann Träger mehrerer, über Spacer gekoppelter Malonsäurederivate sein oder nur ein Malonsäurederivat tragen. Die Anzahl der vom Polymer getragenen Malonsäurederivate richtet sich nach der medizinischen Verwendung und Wirksamkeit des erfindungsgemäßen polyvalenten Malonsäurederivats.

Die kovalente Verknüpfung des Polymers mit dem Spacer bzw. mit einer Verbindung bestehend aus kovalent verküpftem Spacer und Malonsäurederivat erfolgt durch Reaktion zwischen einer reaktiven und einer aktivierten Gruppe. Dabei können sich sowohl die reaktive Gruppe am Ende des Spacers bzw. einer Verbindung bestehend aus kovalent verknüpftem Spacer und Malonsäurederivat und die aktivierte Gruppe auf Seiten des Polymers als auch die aktivierte Gruppe am Ende des Spacers bzw. einer Verbindung aus kovalent verknüpftem Spacer und Malonsäurederivat und die reaktive Gruppe auf Selten des Polymers befinden.

Eine reaktive Gruppe auf Seiten des Malonsäurederivats oder Spacers kann eine OH-, NH₂- oder COOH-Gruppe sein. Eine aktivierte Gruppe auf Seiten des Polymers oder Spacers kann ein Carbamat, z.b. ein Nitrophenylcarbamat, ein Hydroxysuccinimid-Derivat, ein Carbonsäureanhydrid oder ein aus der freien Carbonsäure nach bekannten Methoden aus der Peptidchemie zugänglicher Aktivester sein (z.B. Succinimid-Ester, Hydroxysuccinimid-Ester, gemischtes Anhydrid etc.)

Die Reaktion zwischen reaktiven und aktivierten Gruppen erfolgen nach dem Fachmann bekannten Verfahren zur Alkylierung, Acylierung oder Addition an eine Doppelbindung. Diese Verfahren sind dem Fachmann aus der Literatur bekannt. (Larock, R.C. Comprehensive Organic Transformations, 1989, VCH Verlagsgesellschaft Weinheim).

Ist die aktivierte Gruppe eine Aminogruppe und die reaktive Gruppe eine Carbonsäure, wird die kovalente Verknüpfung durch in der Peptidchemie gängige Aktivierungsmethoden (Carbodiimid, Aktivester, gemischte Anhydride) durchgeführt.

Die erfindungsgemäßen polymerkonjugierten Malonsäurederivate können eine höhere Affinität zu den natürlichen Rezeptoren, beispielsweise zu E- und P-Selektin, als die natürlichen Kohlenhydratliganden haben, wie anhand der nachfolgend beschriebenen Zelladhäsionsassays nachgewiesen werden kann.
1. 96er Mikrotitertestplatten (Nunc Maxisorb®) werden mit 100 µl eines in 50 mM Tris pH 9,5 verdünnten (1 + 100) Ziege anti human IgG Antikörpers (Sigma®) 2 Std. bei Raumtemperatur inkubiert. Nach Entfernen der Antikörperlösung wird einmal mit PBS gewaschen.
2. 150 µl des Blockierungspuffers werden für 1 Std. bei Raumtemperatur in den Näpfchen belassen. Die Zusammensetzung des Blockierungspuffers ist:
   0,1 % Gelatine, 1 % BSA, 5 % Kalbserum, 0,2 mM PMSF, 0,02 %
   Natriumazid. Nach Entfernen des Blockierungspuffers wird einmal mit PBS gewaschen.
3. In die Näpfchen werden je 100 µl Zellkulturüberstand von entsprechend transfektierten und exprimierenden COS-Zellen pipettiert. Die Inkubation erfolgt 2 Std. bei Raumtemperatur. Nach Entfernen des Zellkulturüberstandes wird einmal mit PBS gewaschen.
4. In die Näpfchen werden 20 µl Bindungspuffer gegeben. Der Bindungspuffer hat die Zusammensetzung: 50 mM Hepes, pH 7,5; 100 mM NaCl; 1 mg/ml BSA;
   2 mM MgCl₂; 1mM CaCl₂; 3 mM MnCl₂; 0,02 % Natriumazid; 0,2 mM PMSF. Dazu werden 5 µl der Testsubstanz pipettiert, durch Schwenken der Platte vermischt und 10 Min. bei Raumtempeartur inkubiert.
5. 50 ml einer HL60 Zellkultur mit 200.000 Zellen/ml werden 4 Min. bei 350 g zentrifugiert. Das Pellet wird in 10 ml RPMI 1640 resuspendiert und die Zellen erneut zentrifugiert. Zur Markierung der Zellen werden 50 µg BCECF-AM (Molecular Probes®) in 5 µl wasserfreiem DMSO aufgelöst; anschließend werden 1,5 ml RPMI 1640 auf die BCECF-AM/DMSO-Lösung gegeben. Mit dieser Lösung werden die Zellen resuspendiert und 30 Min. bei 37°C inkubiert. Nach zweiminütiger Zentrifugation bei 350 g wird das markierte Zellpellet in 11 ml Bindungspuffer resuspendiert und die resuspendierten Zellen in 100 µl Aliquots in die Mikrotiterplatten-Näpfchen verteilt. Die Platte wird 10 Min. bei Raumtemperatur stehen gelassen, um die Zellen auf den Boden der Testplatte sedimentieren zu lassen. Dabei haben die Zellen Gelegenheit an das beschichtete Plastik zu adhärieren.
6. Zum Abstoppen des Tests wird die Mikrotiterplatte im 45° Winkel gänzlich in den Stoppuffer getaucht (25 mM Tris, pH 7,5; 125 mM NaCl; 0,1 % BSA; 2 mM MgCl₂; 1 mM CaCl₂; 3 mM MnCl₂; 0,02 % Natriumazid). Durch Invertierung wird der Stoppuffer aus den Näpfchen entfernt und die Prozedur noch zweimal wiederholt.
7. Die Messung der in den Näpfchen festhaftenden, BCECF-AM-markierten Zellen erfolgt in einem Cytofluorimeter (Millipore®), bei einer Empfindlichkeitseinstellung von 4, einer Anregungswellenlänge von 485/22 nm und einer Emissionswellenlänge von 530/25 nm.

Die polymerkonjugierten Malonsäurederivate gemäß der vorliegenden Erfindung stellen allesamt potentielle Liganden für die bisher bekannten Selektine (Proteine, welche auf dem Endothel und anderen Zelloberflächen exprimiert und an spezifische Kohlenhydrat-Liganden binden) dar.

Die erfindungsgemäßen polymerkonjugierten Malonsäurederivate können demzufolge als Anti-Adhäsionstherapeutika eingesetzt werden und verhindern im Fall von Entzündungen, daß die ELAM-1-Rezeptoren auf stimulierten Endothelzellen an Sialyl Lewis-X-Strukturen auf der Oberfläche von Leukozyten binden. Im Fall der Influenza-Therapie verhindern die polymerkonjugierten Malonsäurederivate die Anheftung von Viren an die Neuraminsäure auf der Zelloberfläche und damit auch die Endozytose der Viruspartikel.

Aus diesem Sachverhalt ergibt sich die Möglichkeit zur Prophylaxe, Diagnostik oder Therapie selektinvermittelter Krankheiten. Hierzu gehören beispielsweise:
1. Autoimmunkrankneiten:
   Rheumatische Arthritis, Multiple Sklerose, entzündliche Knochenerkrankungen, Lupus, Myasthenia gravis, Allergien, Osteoarthritis, Asthma, Kontakt-Dermatitis, Psoriasis, Adult respiratory distress syndrome, Transplantatabstoßung.
2. Infektionen:
   Schnupfen, Grippe, Helicobacter pylori, Malaria, Septischer Schock.
3. Krebs:
   Colorectal, Brust, Eierstöcke, Prostata.
4. Zentrales Nervensystem:
   Schlaganfall, Trauma
5. Reperfusionsverletzungen:
   Myocardinfarkt, Angioplastie, instabile Angina, systemischer Schock
6. Weitere:
   Osteroporose, Wunden und schwere Verbrennungen.

Die erfindungsgemäßen Arzneimittel werden im allgemeinen intravenös, oral oder parenteral oder als Implantate verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln. Zäpfchen, Sirupe, Emulsionen, Suspensionen, Aerosole, Tropfen oder injizierbare Lösungen in Ampullenform sowie Präparate mit protrahierte Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z. B. Magnesiumcarbonat, Titandioxid, Laktose, Mannit und andere Zucker, Talkum, Milcheiweiß, Gelatine, Stärke, Vitamine, Cellulose und ihre Derivate, tierische und pflanzliche Öle, Polyethylenglykole und Lösungsmittel, wie etwa steriles Wasser, Alkohole, Glycerin und mehrwertige Alkohole genannt.

Vorzugsweise werden die pharmazeutischen Präparate in Dosierungseinheiten hergestellt und verabreicht. Feste Dosierungseinheiten sind Tabletten, Kapseln und Suppositorien.

Für die Behandlung eines Patienten sind je nach Wirksamkeit der Verbindung, Art der Applikation, Art und Schwere der Erkrankung, Alter und Körpergewicht des Patienten, unterschiedliche Tagesdosen notwendig. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleiner Dosierungseinheiten als auch durch Mehrfachgabe unterteilten Dosen in bestimmten Intervallen erfolgen. Die zu verabreichende Tagesdosis kann außerdem von der Anzahl der während des Krankheitsverlaufs exprimierten Rezeptoren abhängig sein. Es ist vorstellbar, daß im Anfangsstadium der Krankheit nur wenige Rezeptoren auf der Zelloberfläche expirmiert werden und demzufolge die zu verabreichende Tagesdosis geringer ist als bei stark erkrankten Patienten.

Die erfindungsgemäßen Arzneimittel werden dadurch hergestellt, daß man ein polymerkonjugiertes Malonsäurederivat gemäß der vorliegenden Erfindung mit üblichen Träger- sowie gegebenenfalls Zusatz- und/oder Hilfsstoffen in die bzw. eine geeignete Darreichungsform bringt.

Die Verbindung gemäß der vorliegenden Erfindung ist auch zur Herstellung von Antikörpern zur diagnostischen Bestimmung von Liganden geeignet, welche nicht zugänglich, nicht immunogen genug oder unbekannt sind.

Bei vielen Autoimmunerkrankungen und Tumoren sind bestimmte Liganden bzw. Antigene auf der Zellmembran hochreguliert. Diese sind aber häufig nicht bekannt, lassen sich nicht rein isolieren oder sind nicht genug immunogen, um daraus Antikörper herstellen zu können.

Die Verbindung gemäß der vorliegenden Erfindung kann zur Gewinnung von Antikörpern dienen, welche mit Epitopen der natürlichen, unbekannten bzw. nicht zugänglichen Liganden kreuzreagieren. Neben den diagnostischen Anwendungen sind für die auf diese Weise gewonnenen Antikörper auch therapeutische Anwendungen denkbar (A. N. Houghton, D. A. Scheinberg, Semin. Oncol. 13 (1986) 165-179; W. C. Eckelmann, In Vivo Diagnosis and Therapy of Human Tumors with Monoclonal Antibodies; Pergamon Press,London 1988; M. H. Ravindranath, D. L. Morton, R. F. Irie, Cancer Res. 49 (1989) 3891-3897).

Im folgenden werden beispielhaft die Vorschriften für die Synthese eines erfindungsgemäßen polymerkonjugierten Malonsäurederivates beschrieben.

### Beispiel 1

### Synthese von 4,6-lsopropyliden-1,2-didesoxyglucose (1):

Eine Lösung von Tri-O-acetyl-D-glucal (30 g, 110.17 mmol) in Dioxan (400 ml) wird mit Palladiumkohle (10%, 3g) 24 h in einer Wasserstoffatmosphäre hydriert. Die Mischung wird durch Kieselgur filtriert und eingeengt. Zur Abspaltung der Acetylgruppen wird der Rückstand in Methanol (500 ml) aufgenommen und eine 1 M Natriummethanolatlösung (6 ml) zugegeben. Nach 90 min wird mit Amberlite IR-120 neutralisiert, filtriert und im Vakuum eingeengt. Der Rückstand wird mit Toluol (3 x 250 ml) coevaporiert und in DMF (500 ml) aufgenommen. Zu der Lösung gibt man Dimethoxypropan (140 ml, 114,6 mmol) und p-Toluolsulfonsäure (400 mg). Nach 18 h gibt man Triethylamin (3 ml) zu, läßt noch 15 min rühren und engt im Hochvakuum ein. Chromatographie (Toluol/Aceton 4:1) liefert 1d (33 g, 80 %).

¹H-NMR (300 MHz, CDCl₃): δ = 1.41, 1.51 (2s, 6H, 2 CH₃), 1.76 (ddd, 1H, 2-H), 2.0 (ddd, 1H, 2-H), 2.8 (d, 1H, OH), 3.16 (m, 1H, 1-H), 3.46 (dd, 1H, 6-H), 3.53 (m, 1H, 1-H), 3.7 (dd, 1H, 6-H), 3.86 (dd, 1H, 4-H), 3,96 (m, 1H, 5-H).

### Synthese von 4-Allyloxy-1-p-toluolsulfonyloxy-pentan (2):

Eine Mischung aus Pentandiol (21.8 ml, 207 mmol), Allylbromid (11.7 mmol, 138 mmol), Kaliumcarbonat (21 g, 151.8 mmol) und Dibenzo-18-crown-6 wird 24 h im Ultraschallbad behandelt. Anschließend wird mit Dichlormethan (250 ml) verdünnt und 2 x mit Wasser (je 100 ml) gewaschen. Die organische Phase wird über Magnesiumsulfat getrocknet, eingeengt und der Rückstand mit Pyridin (50 ml, 600 mmol), Dichlormethan (700 ml) und p-Toluolsulfonsäurechlorid (40 g, 207 mmol) gerührt. Nach 16 h wird mit gesättigter Kochsalzlösung gewaschen, die organische Phase getrocknet und eingeengt.
Flash-Chromatographie (Hexan/Essigester 6:1 → 5:1) liefert Verbindung 2 (19.9g, 53%)
¹H-NMR (300 MHz, CDCl₃): δ = 1.39, 1.53, 1.66 (3m, 6H, -CH₂-CH₂-CH₂-), 2.45 (s, 3 H, CH₃ₜₒₛ), 3.38 (t, 2H, OCH₂-), 3.93 (m, 2H, O-CH₂-CH=CH₂), 4.01 (t, 2H, OCH₂-), 5.20 (m, 2 H, O-CH₂-CH=CH₂), 5.88 (m, 1H, O-CH₂-CH=CH₂), 7.34, 7.78 (2 m, 4H, Tosyl-Hₐᵣₒₘₐₜ).

### Synthese von 4-Allyloxy-1-hydroxybutyl-(1→3)-1,2-didesoxyglucose (3):

Zu einer Lösung aus 1 (10 g, 53.47 mmol) in Dimethylformamid (100 ml) gibt man Natriumhydrid (1.56 g, 65 mmol). Nach 30 min tropft man eine Lösung von 2 (19 g, 63.8 mmol) zu. Nach 18 h gibt man Wasser (5 ml) zu, verdünnt mit Ether und wäscht die organische Phase mit Wasser und engt sie anschließend ein. Der Rückstand wird mit Tetrahydrofuran (150 ml) und 1 M Salzsäure (5 ml) aufgenommen und auf 60 °C erwärmt. Nach 90 min wird im Vakuum eingeengt, mit Toluol coevaporiert und der Rückstand mit Hexan/Essigester 1:1 → 1:3 chromatografiert. Man erhält 3 (12.1 g, 83%). ¹H-NMR (300 MHz, CDCl₃): δ = 2.02 (m, 1 H, 2-H), 2.58 (bt, 1H, OH), 3.93 (m, 2H, O-CH₂-CH = CH₂); 5.22 (m, 2 H, O-CH₂-CH = CH₂), 5.92 (m, 1H, O-CH₂-CH = CH₂).

### Synthese von 4-Allyloxy-1-hydroxybutyl-(1→3)-6-O-p-toluolsulfonyl-1,2-didesoxyglucose (4):

Zu einer eiskalten Lösung von 3 (19 g, 69.3 mmol) in Pyridin (100 ml) gibt man Tosylchlorid (15.9 g, 83.2 mmol). Nach 18 h bei 0 °C wird im Vkuum eingeengt, mit Toluol coevaporiert und der Rückstand mit Ether aufgenommen und mit Wasser gewaschen. Die organische Phase wird über Natriumsulfat getrocknet, eingeengt und chromatografiert (Hexan/Essigester 3:1). Ausbeute:
28.5 g. (96%).
¹H-NMR (300 MHz, CDCl₃): δ = ¹H-NMR (300 MHz, CDCl₃): δ = 2.02 (m, 1 H, 2-H), 2.4 (s, 3H, CH₃), 2.73 (bs, 1H, 4-OH), 3.95 (m, 2H, O-CH₂-CH=CH₂), 5.22 (m, 2 H, O-CH₂-CH=CH₂), 5.9 (m, 1H, O-CH₂-CH=CH₂), 7.32, 7.80 (2m, 4H, Hₜₒₛ).

### Synthese von 4-Allyloxy-1-hydroxybutyl-1→3)-2-azido-1-hydroxyethyl-(1→6)-1,2-didesoxyglucose (5):

Zu einer Lösung aus Azidoethanol (5 g, 57.5 mmol) in Dimethylformamid (100 ml) gibt man Natriumhydrid (1.56 g, 65 mmol). Nach 30 min tropft man eine Lösung von 4 (4.93 g, 11.5 mmol) zu. Nach 18 h gibt man Wasser (5 ml) zu, verdünnt mit Ether und wäscht die organische Phase mit Wasser und engt sie anschließend ein. Der Rückstand wird chromatografisch (Hexan/Essigester 2:1 1:1) gereinigt. Ausbeute: 3.58 g (91%).
¹H-NMR (300 MHz, CDCl₃): δ = 2.02 (m. 1 H, 2-H), 2.78 (d, 1H, 4-OH), 3.96 (m, 2H, O-CH₂-CH=CH₂), 5.22 (m, 2 H, O-CH₂-CH = CH₂), 5.9 (m, 1H, O-CH₂-CH = CH₂).

### Synthese von 4-Allyloxy-1-hydroxybutyl-(1→3)-[(2,3,4-tri-O-benzyl-α-Lfucopyranosyl)-(1→4)]-2-azido-1-hydroxyethyl-(1→6)-1,2-didesoxyglucose (6) :

Zu einer Lösung von 5 (1.76 g, 5.13 mmol) in Ether (30 ml) gibt man eine 0.1 M Trimethylsilyltrifluormethansulfonat-Lösung (0.51 ml) und tropft anschließend eine Lösung von O-(2,3,4-Tri-O-benzyl-L-fucopyranosyl)-trichloracetimidat binnen 15 min dazu. Nach weiteren 15 min wird mit Natriumhydrogencarbonat (0.5 g) neutralisiert, filtriert und eingeengt. Chromatographie (Dichlormethan/Methanol 80:1) liefert 6 (3.43 g, 88%).

### Synthese von 1,4-Dihydroxybutyl-(1→3)-[(2,3,4-tri-O-benzyl-α-L-fucopyranosyl)-(1→4)]-2-azido-1-hydroxyethyl-(1→6)-1,2-didesoxyglucose (7):

Zu einer Lösung aus 6 (3.43 g, 4.52 mmol) in Tetrahydrofuran (34 ml) gibt man eine Lösung aus vorhydriertem Bis(methyldiphenylphosphin)-cyclooctadieniridium-I-hexafluorophosphat (52 mg, 0.06 mmol) in THF (5 ml). Nach 90 min gibt man Wasser (2 ml) und lod (1.076 g) dazu und rührt weitere 2 h. Es wird mit Dichlormethan verdünnt, mit einer 20%igen Natriumthiosulfatlösung und anschließend Wasser gewaschen. Der nach dem Einengen erhaltene Rückstand wird chromatographiert (Hexan/Essigester 1:1 → 2:1). Man erhält Verbindung 7 (2.21 g, 68%).

### Synthese von 4-Toxyloxy-1-hydroxybutyl-(1→3)-[(2,3,4-tri-O-benzyl-α-Lfucopyranosyl-(1→4)]-2-azido-1-hydroxyethyl-(1→6)-1,2-didesoxyglucose (8):

Zu einer eiskalten Lösung von 7 (618 mg, 0.86 mmol) in Pyridin (5 ml) gibt man Tosylchlorid (212 mg, 1.11 mmol). Nach 24 h wird im Vakuum eingeengt, mit Toluol coevaporiert, der Rückstand in Ether aufgenommen und mit Wasser gewaschen. Die organische Phase wird über Natriumsulfat getrocknet, filtriert und eingeengt. Chromatographie (Hexan/Essigester 3:1 2:1) liefert Verbindung 8 (533 mg, 71%).
¹H-NMR (300 MHz, CDCl₃): δ = 1.09 (d, 3H, 6-H_{fuc}), 2.43 (s, 3 H, CH₃), 5.05 (d, 1H, 1-H).

### Synthese von 4-Dimethylmalonyl-1-hydroxybutyl-(1→3)-[(2,3,4-tri-O-benzyl-α-Lfucopyranosyl)-(1→4)]-2-azido-1-hydroxyethyl-(1→6)-1,2-didesoxyglucose (8):

Eine Mischung aus 8 (986 mg, 1.13 mmol), Malonsäuredimethylester (17 ml), Kaliumcarbonat (1.08 g) und Dibenzo-18-crown-6 wird 1 h bei 100°C gerührt. Man verdünnt mit Essigester, filtriert über Kieselgel und engt den Rückstand im Hochvakuum bei 70 °C ein. Chromatographie (Hexan/Essigester 3:12:1) liefert 8 (684 mg, 73%).
¹H-NMR (300 MHz, CDCl3): δ = 1.12 (d, 3H, 6-H_{fuc}), 3.72 (2s, 6H, 2CH₃), 5.05 (d, 1H, 1-H).

### Synthese von 4-Malonyl-1-hydroxybutyl-(1→3)-[α-L-fucopyranosyl-(1→4)]-2-amino-1-hydroxyethyl-(1→6)-1,2-didesoxyglucose (9):

Zu einer Lösung aus 8 (506 mg, 0.608 mmol) in Methanol gibt man succesiv soviel 2 M Natronlauge, daß gerade nichts ausfällt. Nach vollständiger Verseifung wird mit Amberlite® IR-120 neutralisiert, filtriert und eingeengt. Der Rückstand wird mit Dioxan/Wasser (9 : 1, 15 ml) aufgenommen, mit Palladiumkohle (10%, 400 mg) versetzt und 18 h in einer Wasserstoffatmosphäre hydriert. Filtration und Chromatographie mittels Biogel® P2 liefern 9 (281 mg, 91%).
¹H-NMR (300 MHz, D₂O): δ = 1.06 (d, 3H, 6-H_{fuc}), 1.18 (m, 4H, 2 CH₂), 1.45 (2m, 3H, 1-H, CH₂), 1.60 (m, 2H, CH₂), 2.14 (m, 1H, 1-H), 4.26 (q, 1H, 5-H_{fuc}), 4.74 (d, 1H, 1H_{fuc})

### Beispiel 2

### Poly-D,L-succinimid-co-α,β-(5-Carboxypentyl)-D,L-aspartamid

500 mg PSI (MG 9 600) werden in 2 ml DMF gelöst und mit 1.38 g 6-Aminohexansäure, gelöst in 8 ml Formamid, und 1 ml Triethylamin versetzt. Es wird 13 h bei 45°C gerührt und anschließend mit 1-Butanol gefällt. Nach Waschen des Polymers mit Methanol wird in H2O aufgenommen und gefriergetrocknet.
Ausbeute: 350 mg
Substitutionsgrad laut NMR: 8% Aminohexansäure

### Beispiel 3

### Poly-D,L-succinimid-co-α,β-(5-Carboxypentyl)-D,L-aspartamid

1g PSI (MG 24 000) werden in 4 ml DMF gelöst und mit 2.1 g 6-Aminohexansäure, gelöst in 10 ml Formamid, und 25 mg DMAP versetzt. Es wird 3 d bei Raumtemperatur und 9 h bei 45°C gerührt und anschließend mit 1-Butanol gefällt. Nach Waschen des Polymers mit Methanol wird in H₂O aufgenommen und gefriergetrocknet.
Ausbeute: 980 mg
Substitutionsgrad laut NMR: 18% Aminohexansäure

### Beispiel 4

### Poly-D,L-succinimid-co-α,β-(2-Carboxyethyl)-D,L-aspartamid

5 g PSI (MG 9 600) werden in 20 ml DMF gelöst und mit 7.5 g β-Alanin, gelöst in 50 ml Formamid, und 100 mg DMAP versetzt. Es wird 3 d bei Raumtemperatur und 9 h bei 45°C gerührt und anschließend mit 1-Butanol gefällt. Nach Waschen des Polymers mit Methanol wird in H₂O aufgenommen und gefriergetrocknet.
Ausbeute: 3.9 g
Substitutionsgrad laut NMR: 14% β-Alanin

### Beispiel 5

### Poly-D,L-succinimid-co-α,β-(2-(2-hydroxymethyl)-carboxyethyl)-D,L-aspartamid

1 g PSI (MG 9 600) werden in 4 ml DMF gelöst und mit 1.5 g L-Serin, gelöst in 8 ml Formamid, und 4 ml Triethylamin versetzt. Es wird 3 d bei Raumtemperatur und 9 h bei 45°C gerührt und anschließend mit 1-Butanol gefällt. Nach Waschen des Polymers mit Methanol wird in H₂O aufgenommen und gefriergetrocknet.
Ausbeute: 1 g
Substitutionsgrad laut NMR: 14% L-Serin

### Beispiel 6

### Kopplung des Polymers aus Beispiel 2 mit Verbindung 9 (aus Beispiel 1)

100 mg PCPA aus Beispiel 2 werden in 2 ml H₂O gelöst und mit 20 mg Verbindung 9 (aus Beispiel 1) versetzt. Während 36 h werden 4 mal je 20 mg 1-Ethyl-3-(3-Dimethylaminopropyl)-carbodiimid-Hydrochlorid (EDC) zugegeben. Nach Dialyse und Gefriertrocknung verbleibt das Produkt.
Ausbeute: 112 mg
Substitutionsgrad laut NMR: 5 % Verbindung 9

### Beispiel 7

### Kopplung des Polymers aus Beispiel 3 mit Verbindung 9 (aus Beispiel 1)

100 mg PCPA aus Beispiel 3 werden in 2 ml H₂O gelöst und mit 20 mg Verbindung 9 (aus Beispiel 1) versetzt. Während 36 h werden 4 mal je 20 mg 1-Ethyl-3-(3-Dimethylaminopropyl)- carbodiimid-Hydrochlorid (EDC) zugegeben. Nach Dialyse und Gefriertrocknung verbleibt das Produkt.
Ausbeute: 90 mg
Substitutionsgrad laut NMR: 12 % Verbindung 9

## Patentansprüche

1. Verbindung bestehend aus einer Polyaminosäure, welche über jeweils einen Spacer der Formel I,
-[-Q¹-(CH₂)ₚ-Q²-]- I,
worin
Q¹ -CH₂- oder 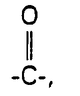
Q² -NH- oder 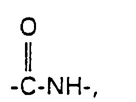
p eine Zahl von 1 bis 6 bedeuten,
mit wenigstens einem Rest der Formel II 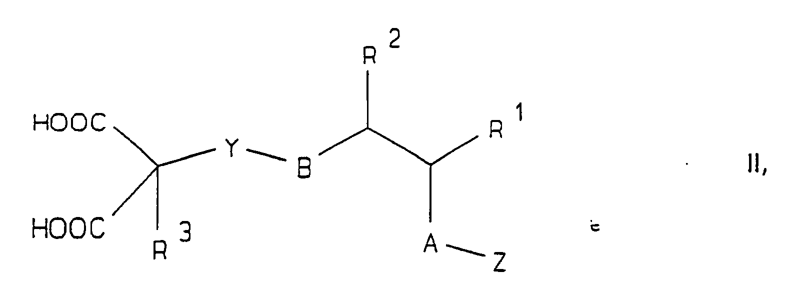 in der
R¹ und R² zu einem sechsgliedrigen Carbo- oder Heterocyclus mit mindestens einem der Ringsubstituenten R⁴, R⁵ und R⁶ gehören und
R³ H, (CH₂)ₘX oder CH₂O(CH₂)ₘX¹ bedeutet,
A und B unabhängig voneinander O, S, NH, HN-CO, OC-NH, O-CO, OC-O, NH-CO-O, O-CO-NH, S-CO, SC-O, O-CS-S, S-CS-O, NH-CS-S, S-CS-NH oder CH₂ bedeuten und
Z eine Pyranose, eine Furanose, einen offenkettigen Polyalkohol darstellt oder Y-X⁶ bedeutet,
Y -O-(CX²X³)ₙ, -(CX²X³)ₙ, -CH₂-(CX²X³)⁻ₙ wobei
R⁴, R⁵ und R⁶ unabhängig voneinander H, OH, -O-(CH₂)_{q}X⁴, CH₂O(CH₂)_{q}X⁵ oder HNC(O)CH₃ und
X, X¹, X² und X³ unabhängig voneinander H, NH₂, COOH, OH, CH₂OH, CH₂NH₂, C₁-C₂₀-Alkyl oder C₆-C₁₀-Aryl,
X⁴ und X⁵ unabhängig voneinander -NH- oder -O- und
X⁶ OH oder 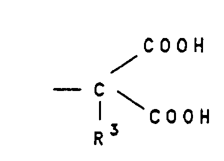 und
m, n und q unabhängig voneinander eine Zahl von 1 bis 20 bedeuten,
über einen der Ringsubstituenten R⁴, R⁵ oder R⁶ verknüpft ist. Wobei der Belegunsgrad der Polyaminosäure mit dem Rest gemäß Formel II via Spacer zwischen 0,5 und 50Mol % liegt.

2. Verbindung nach Anspruch 1, **dadurch gekennzeichnet, daß** die Polyaminosäure Polysuccinimid ist.

3. Verbindung nach Anspruch 1, **dadurch gekennzeichnet, daß** die Polyaminosäure Polyaspartamid ist.

4. Verbindung nach Anspruch 1, **dadurch gekennzeichnet, daß** die Polyaminosäure Polyglutamat ist.

5. Verbindung nach Anspruch 1, **dadurch gekennzeichnet, daß** die Polyaminosäure Polylysinfumaramid ist.

6. Verbindung nach Anspruch 1, **dadurch gekennzeichnet, daß** die Polyaminosäure ein Copolymer aus Polysuccinimid und Polyaspartamid ist

7. Verbindung nach Anspruch 1, **dadurch gekennzeichnet, daß** die Polyaminosäure ein Copolymer aus Polyhydroxyethylaspartamid und Polyaspartamid ist.

8. Verbindung nach Anspruch 1, **dadurch gekennzeichnet, daß** die Polyaminosäure ein Copolymer aus Polysuccinimid, Polyaspartamid und Polyhydroxyethylaspartamid ist.

9. Verbindung nach einem der Ansprüche 1 bis 8, **dadurch gekennzeichnet, daß** R1 und R2 zu einem substituierten Tetrahydropyranring gehören,
A und B O,
Z eine -Fucopyranosyl-Gruppe,
Y (CX²X³)ₙ bedeuten, wobei
R³, X² und X³ H bedeuten und n für 5 steht.

10. Verbindung nach Anspruch 9, **dadurch gekennzeichnet, daß** die Substituenten des Tetrahydropyranrings R⁴ und R⁵ jeweils H, der im Tetrahydropyranring zwischen dem Ringheteroatom O und dem Ringsubstituenten -A-Z liegende Substituent
R⁶ -CH₂O(CH₂)_{q}X⁵ und
X⁵ -NH- bedeuten.

11. Verbindung nach Anspruch 10, **dadurch gekennzeichnet, daß** q für 2 steht.

12. Verbindung nach Anspruch 9, **dadurch gekennzeichnet, daß** der Tetrahydropyranring eine Pyranose ist.

13. Verbindung nach Anspruch 12, **dadurch gekennzeichnet, daß** die Pyranose N-Acetyl-D-glucosamin ist.

14. Verbindung nach einem der Ansprüche 1 bis 8, **dadurch gekennzeichnet, daß**
R¹ und R² zu einem sechsgliedrigen Carbocyclus gehören,
A-Z 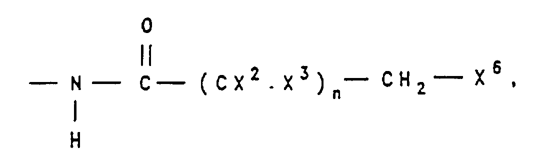
-B-Y- 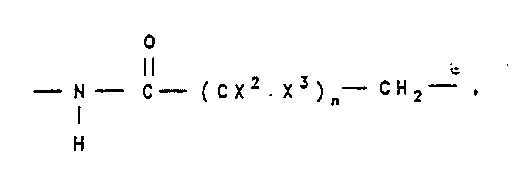
R³ und X² H,
X³ und X⁶ OH und
n 4 bedeuten.

15. Verbindung nach Anspruch 14, **dadurch gekennzeichnet, daß**
X⁶ 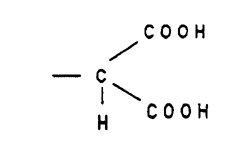 **bedeutet**.

16. Verfahren zur Herstellung einer Verbindung gemäß einem der Ansprüche 1 bis 15, **dadurch gekennzeichnet, daß** die kovalente Verknüpfung des Polymers mit einer Verbindung der Formel III 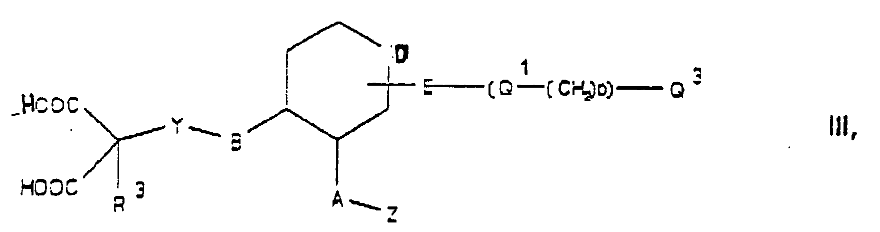 worin
D -NH-, -O- oder -CH₂-,
E -O(CH₂)_{q}NH-, -O(CH₂)_{q}O-, -CH₂O(CH₂)_{q}NH- oder -CH₂O(CH₂)_{q}O- und
Q³ -OH, -NH₂ oder 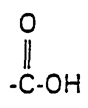 bedeuten und
die übrigen Variablen die in Anspruch 1 genannten Bedeutungen haben, durch Reaktion zwischen einer reaktiven und einer aktivierten Gruppe erfolgt.

17. Arzneimittel, enthaltend eine Verbindung gemäß einem der Ansprüche 1 bis 15 und gegebenfalls pharmazeutische Träger.

18. Verwendung einer Verbindung gemäß einem der Ansprüche 1 bis 15 zur Herstellung eines Arzneimittels zur Therapie oder Prophylaxe von Krankheiten, bei welchen bakterielle oder virale Infektionen, entzündliche Prozesse oder metastasierende Tumoren involviert sind.

19. Verwendung einer Verbindung gemäß einem der Ansprüche 1 bis 15 zur Herstellung eines Mittels zur Diagnose von Krankheiten, bei welchen bakterielle oder virale Infektionen, entzündliche Prozesse oder metastasierende Tumoren involviert sind.

## Claims

1. A compound consisting of a polyamino acid which via a respective spacer of formula I,
-[-Q¹-(CH₂)ₚ-Q²-]- I,
wherein
Q¹ is -CH₂- or 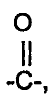
Q² is -NH- or 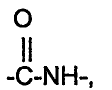
p is a number of 1 to 6,
is linked with at least a residue of formula II 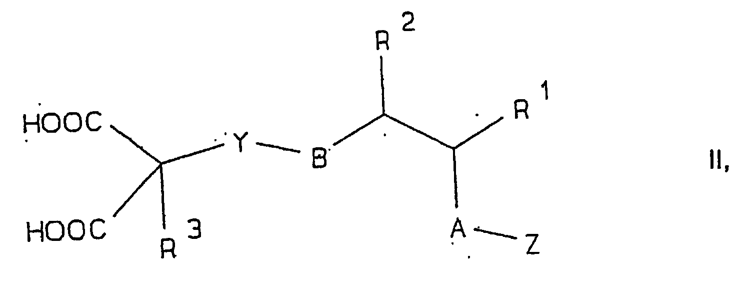 in which
R¹ and R² belong to a six-membered carbo- or heterocycle with at least one of the ring substituents R⁴, R⁵, and R⁶, and
R³ is H, (CH₂)ₘX or CH₂O(CH₂)ₘX¹,
A and B are, independently of each other, O, S, NH, HN-CO, OC-NH, O-CO, OC-O, NH-CO-O, O-CO-NH, S-CO, SC-O, O-CS-S, S-CS-O, NH-CS-S, S-CS-HN, or CH₂, and
Z represents a pyranose, a furanose, an open-chain poly-alcohol, or means Y-X⁶,
Y -O-(CX²X³)ₙ, -(CX²X³)ₙ, -CH₂-(CX²X²)⁻ₙ wherein
R⁴, R⁵ and R⁶ are, independently of one another, H, OH, -O-(CH₂)_{q}X⁴, CH₂O(CH₂)_{q}X⁵ or HNC(O)CH₃, and
X, X¹, X² and X³ are, independently of one another, H, NH₂, COOH, OH, CH₂OH, CH₂NH₂, C₁-C₂₀ alkyl or C₆-C₁₀ aryl,
X⁴ and X⁵ are, independently of each other, -NH- or -O-, and
X⁶ OH or 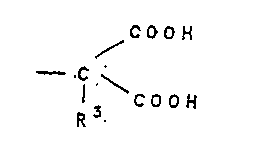 and
m, n and q are, independently of one another, a number of 1 to 20,
via one of the ring substituents R⁴, R⁵ or R⁶, wherein the degree of coverage of said polyamino acid with the residue according to formula II via spacer is between 0.5 and 50 mol%.

2. The compound according to claim 1, **characterized in that** said polyamino acid is polysuccinimide.

3. The compound according to claim 1, **characterized in that** said polyamino acid is polyaspartamide.

4. The compound according to claim 1, **characterized in that** said polyamino acid is polyglutamate.

5. The compound according to claim 1, **characterized in that** said polyamino acid is polylysine fumaramide.

6. The compound according to claim 1, **characterized in that** said polyamino acid is a copolymer of polysuccinimide and polyaspartamide.

7. The compound according to claim 1, **characterized in that** said polyamino acid is a copolymer of polyhydroxyethyl aspartamide and polyaspartamide.

8. The compound according to claim 1, **characterized in that** said polyamino acid is a copolymer of polysuccinimide, polyaspartamide and polyhydroxyethyl aspartamide.

9. The compound according to any one of claims 1 to 8, **characterized in that** R1 and R2 belong to a substituted tetrahydropyrane ring,
A and B are O,
Z is a -fucopyranosyl group,
Y is (CX²X³)ₙ, wherein
R³, X² and X³ mean H, and n stands for 5.

10. The compound according to claim 9, **characterized in that** the substituents of the tetrahydropyrane ring R⁴ and R⁵ are each H, the substituent lying in the tetrahydropyrane ring between the ring heteroatom O and the ring substituent -A-Z
R⁶ is -CH₂O(CH₂)_{q}X⁵, and
X⁵ is -NH-.

11. The compound according to claim 10, **characterized in that** q stands for 2.

12. The compound according to claim 9, **characterized in that** the tetrahydropyrane ring is a pyranose.

13. The compound according to claim 12, **characterized in that** the pyranose is N-acetyl-D-glucosamine.

14. The compound according to any one of claims 1 to 8, **characterized in that**
R¹ and R² belong to a six-membered carbocycle,
A-Z is 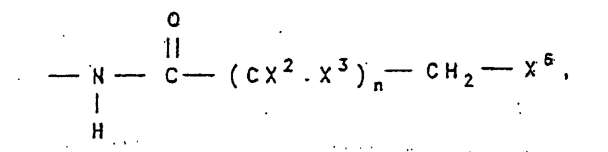
-B-Y- 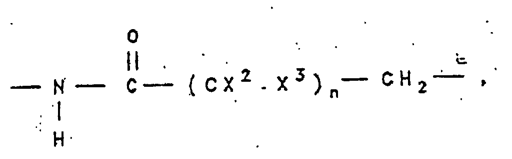
R³ and X² are H,
X³ and X⁶ are OH, and
n is 4.

15. The compound according to claim 14, **characterized in that**
X⁶ is

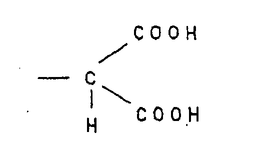

16. A method for preparing a compound according to any one of claims 1 to 15, **characterized in that** the covalent linkage of the polymer with a compound of formula III 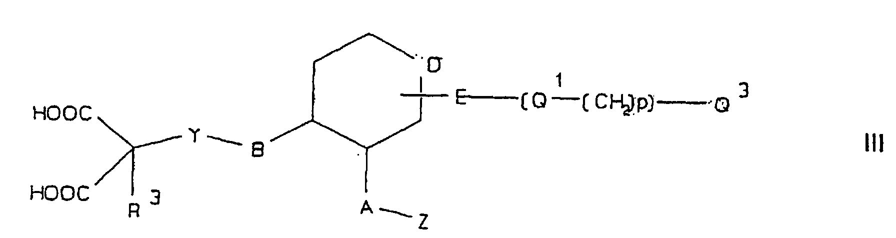 wherein
D is -NH-, -O-, or -CH₂-,
E -O(CH₂)_{q}NH-, -O(CH₂)_{q}O-, -CH₂O(CH₂)_{q}NH- or -CH₂O(CH₂)_{q}O-, and
Q3 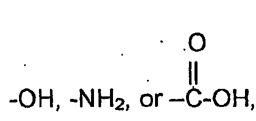 and
the remaining variables have the meanings indicated in claim 1, is carried out by reaction between a reactive and an activated group.

17. A medicament containing a compound according to any one of claims 1 to 15 and, optionally, pharmaceutical carriers.

18. Use of a compound according to any one of claims 1 to 15 for preparing a medicament for the treatment or prophylaxis of diseases in which bacterial or viral infections, inflammatory processes or metastasizing tumors are involved.

19. Use of a compound according to any one of claims 1 to 15 for preparing an agent for diagnosing diseases in which bacterial or viral infections, inflammatory processes or metastasizing tumors are involved.

## Revendications

1. Composé consistant en un polyaminoacide qui est lié, dans chaque cas par le biais d'un espaceur de formule I,
-[-Q¹-(CH₂)ₚ-Q²-]- I
où
Q¹ représente -CH₂- ou 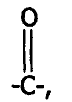
Q² représente -NH- ou 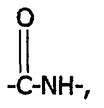
p représente un nombre de 1 à 6,
avec au moins un reste de formule II 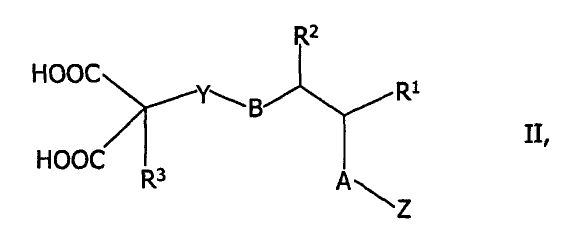 où
R¹ et R² appartiennent à un carbocycle ou hétérocycle à 6 chaînons comportant au moins l'un des substituants du cycle R⁴, R⁵ et R⁶ et
R³ représente H, (CH₂)ₘX ou CH₂O(CH₂)ₘX¹,
A et B représentent indépendamment l'un de l'autre O, S, NH, HN-CO, OC-NH, O-CO, OC-O, NH-CO-O, O-CO-NH, S-CO, SC-O, O-CS-S, S-CS-O, NH-CS-S, S-CS-NH ou CH₂ et
Z représente un pyranose, un furanose, un polyalcool à chaîne ouverte ou Y-X⁶,
Y représente -O-(CX²X³)ₙ, -(CX²X³)ₙ, -CH₂-(CX²X³)⁻ₙ où
R⁴, R⁵ et R⁶ représentent indépendamment les uns des autres H, OH, -O-(CH₂)_{q}X⁴, CH₂O(CH₂)_{q}X⁵ ou HNC(O)CH₃ et
X, X¹, X² et X³ représentent indépendamment les uns des autres H, NH₂, COOH, OH, CH₂OH, CH₂NH₂, alkyle en C₁-C₂₀ ou aryle en C₆-C₁₀,
X⁴ et X⁵ représentent indépendamment l'un de l'autre -NH- ou -O- et
X⁶ représente OH ou 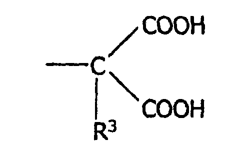 et
m, n et q représentent indépendamment les uns des autres un nombre de 1 à 20,
par l'intermédiaire de l'un des substituants du cycle R⁴, R⁵ et R⁶, où le degré de recouvrement du polyaminoacide avec le reste de formule II par le biais de l'espaceur est situé entre 0,5 et 50 mol%.

2. Composé selon la revendication 1, **caractérisé en ce que** le polyaminoacide est le polysuccinimide.

3. Composé selon la revendication 1, **caractérisé en ce que** le polyaminoacide est le polyaspartamide.

4. Composé selon la revendication 1, **caractérisé en ce que** le polyaminoacide est le polyglutamate.

5. Composé selon la revendication 1, **caractérisé en ce que** le polyaminoacide est le polylysinefumaramide.

6. Composé selon la revendication 1, **caractérisé en ce que** le polyaminoacide est un copolymère de polysuccinimide et de polyaspartamide.

7. Composé selon la revendication 1, **caractérisé en ce que** le polyaminoacide est un copolymère de polyhydroxyéthylaspartamide et de polyaspartamide.

8. Composé selon la revendication 1, **caractérisé en ce que** le polyaminoacide est un copolymère de polysuccinimide, de polyaspartamide et de polyhydroxyéthylaspartamide.

9. Composé selon l'une des revendications 1 à 8, **caractérisé en ce que** R¹ et R² font partie d'un cycle tétrahydropyrane substitué,
A et B représentent O,
Z représente un groupe fucopyranosyle,
Y représente (CX²X³)ₙ, où
R³, X² et X³ représentent H et n représente 5.

10. Composé selon la revendication 9, **caractérisé en ce que** les substituants du cycle tétrahydropyrane R⁴ et R⁵ représentent chacun H, le substituant situé dans le cycle tétrahydropyrane entre l'hétéroatome du cycle O et le substituant du cycle -A-Z
R⁶ représente -CH₂O(CH₂)_{q}X⁵ et
X⁵ représente -NH-.

11. Composé selon la revendication 10, **caractérisé en ce que** q représente 2.

12. Composé selon la revendication 9, **caractérisé en ce que** le cycle tétrahydropyrane est un pyranose.

13. Composé selon la revendication 12, **caractérisé en ce que** le pyranose est la N-acétyl-D-giucosamine.

14. Composé selon l'une des revendications 1 à 8, **caractérisé en ce que**
R¹ et R² font partie d'un carbocycle à 6 chaînons,
A-Z représente 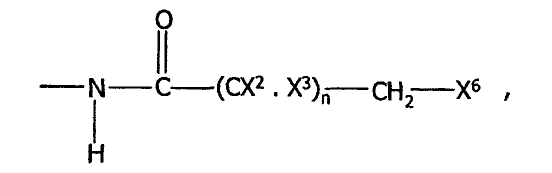 -B-Y- représente 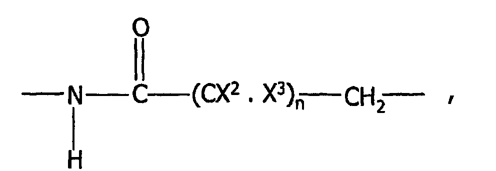 R³ et X² représentent H,
X³ et X⁶ représentent OH et
n représente 4.

15. Composé selon la revendication 14, **caractérisé en ce que** X⁶ représente

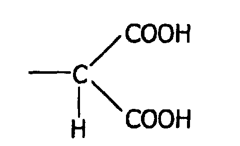

16. Procédé de préparation d'un composé selon l'une des revendications 1 à 15, **caractérisé en ce que** la liaison covalente du polymère avec un composé de formule III 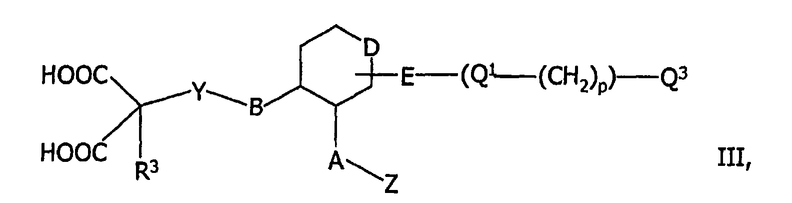 où
D représente -NH-, -O- ou -CH₂-,
E représente -O(CH₂)_{q}NH-, -O(CH₂)_{q}O-, -CH₂O(CH₂)_{q}NH- ou -CH₂O(CH₂)_{q}O- et
Q³ représente -OH, -NH₂ ou 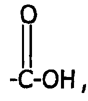 et
les autres variables ont les significations citées dans la revendication 1, a lieu par réaction entre un groupe réactif et un groupe activé.

17. Médicament contenant un composé selon l'une des revendications 1 à 15 et éventuellement des supports pharmaceutiques.

18. Utilisation d'un composé selon l'une des revendications 1 à 15 pour la fabrication d'un médicament pour la thérapie ou la prophylaxie de maladies dans lesquelles des infections bactériennes ou virales, des processus inflammatoires ou des tumeurs métastasantes sont impliqués.

19. Utilisation d'un composé selon l'une des revendications 1 à 15 pour la fabrication d'un agent pour le diagnostic de maladies dans lesquelles les infections bactériennes ou virales, des processus inflammatoires ou des tumeurs métastasantes sont impliqués.
